# EUROPÄISCHE PATENTANMELDUNG

(11) **EP 2 444 056 A1**
(43) Veröffentlichungstag der Anmeldung: **25.04.2012**
(21) Anmeldenummer: 11175274.7
(22) Anmeldetag: 26.07.2011
(51) Int. Cl.: A61K 8/03, A61K 8/891, A61Q 5/12, A61K 8/58

(54) **Sprühbares zweiphasiges Haarkonditioniermittel**

(30) Priorität: 19.08.2010 DE 102010039535
(71) Anmelder: Henkel AG & Co. KGaA, 40589 Düsseldorf (DE)
(72) Erfinder: Battermann, Marlene, 21271 Asendorf (DE); Westphal, Petra, 21629 Neu Wulmstorf (DE); Delowsky, Jens, 22844 Norderstedt (DE)

(57) **Zusammenfassung**

Die Erfindung betrifft flüssige zweiphasige Haarkonditioniermittel, die den Haaren vorteilhafte Eigenschaften verleihen, enthaltend - bezogen auf das Gewicht des anwendungsbereiten Mittels
a) 30 bis 95 Gew.-% einer transparenten hydrophilen Phase, die - bezogen auf ihr Gewicht - mindestens 40 Gew.-% Wasser enthält, und
b) 5 bis 70 Gew.-% einer von a) durch eine Phasengrenze getrennt vorliegenden transparenten Ölphase, die - bezogen auf ihr Gewicht - mindestens 5 Gew.-% eines flüchtigen linearen Silikons enthält.

## Beschreibung

Die Erfindung liegt auf dem Gebiet der kosmetischen Mittel und betrifft ein flüssiges zweiphasiges Haarkonditioniermittel, das vorzugsweise versprüht werden kann, sowie ein Verfahren zur Haarkonditionierung unter Verwendung des Mittels.

Sprühbare Haarkonditioniermittel sind aus dem Stand der Technik bekannt. Sie werden als feiner Sprühnebel auf die Haare aufgebracht und haben den Vorteil, dass sie neben einem einheitlichen Sprühbild eine sehr gute Verteilbarkeit des Haarkonditioniermittels auf den Haaren gewährleisten. Üblicherweise werden solche Produkte in transparenten Behältnissen angeboten, die zwei sichtbar voneinander getrennte Phasen - eine wässrige Phase und eine ölhaltige Phase - enthalten. Unmittelbar vor der Anwendung werden die beiden Phasen durch Schütteln kurzzeitig miteinander vermischt. Nach der Anwendung sollen sich die Phasen schnell wieder separieren.

Problematisch bei der Herstellung solcher Produkte ist die Stabilisierung der Phasen, d.h. die schnelle Separierung der Phasen nach der Anwendung. Weiterhin sollen die Phasen nach der Separierung keine unerwünschten Trübungen an der Phasengrenze aufweisen. Eine solche Trübung kann beispielsweise durch eine partielle Emulgierung und/oder Dispergierung der einen Phase in der anderen auftreten.

Für die schnelle Phasentrennung wurden bisher Kohlenwasserstoffe und/oder Cyclomethicone eingesetzt. Diese Stoffe begegnen zunehmend ökologischen Bedenken.

Von den Verbrauchern werden transparente zweiphasige Haarkonditioniermittel bevorzugt, weil mit klaren Mitteln Reinheit und Pflege assoziiert werden.

Weiterhin bevorzugen die Verbraucher Haarkonditioniermittel, die überwiegend wässrig sind und keine Inhaltsstoffe enthalten, die für die Umwelt schädlich sein könnten.

In der WO 2010/069995 werden klare, zweiphasige Zusammensetzungen für die topische Anwendung - beispielsweise die Haarpflege - offenbart. Diese Zusammensetzungen basieren auf einer hydrophilen Phase, die überwiegend Polyole enthält, sowie einer Ölphase, die bevorzugt keine Silikone enthält. Bedingt durch den hohen Polyolgehalt in der hydrophilen Phase und einen höheren Gehalt an Kohlenwasserstoffen in der Ölphase zeigen sie eine schnelle Separation der Phasen nach der Durchmischung.

Ziel der vorliegenden Erfindung war es, transparente, zweiphasige Haarkonditioniermittel herzustellen, deren Phasen sich durch Schütteln durchmischen lassen und im Ruhezustand schnell wieder in zwei transparente Phasen separieren, und die vorzugsweise versprüht werden können.

Ein weiteres Ziel der vorliegenden Erfindung war es transparente, zweiphasige Haarkonditioniermittel mit hohem Haarpflegevermögen herzustellen, die eine überwiegend wässrige hydrophile Phase umfassen.

Die Separierung der Phasen soll aufgrund ökologischer Gesichtspunkte bevorzugt ohne den Einsatz von Cyclomethiconen und/oder Kohlenwasserstoffen in der Ölphase möglich sein.

Vorgeschlagen werden daher flüssige transparente zweiphasige Haarkonditioniermittel, die sich versprühen lassen und durch einen höheren Gehalt an flüchtigen linearen Silikonen in der Ölphase eine effektive Trennung der transparenten Phasen vor und nach deren Durchmischung aufweisen.

Gegenstand der vorliegenden Erfindung sind daher flüssige zweiphasige Haarkonditioniermittel, enthaltend - bezogen auf das Gewicht des anwendungsbereiten Mittels -
a) 30 bis 95 Gew.-% einer transparenten hydrophilen Phase, die - bezogen auf ihr Gewicht - mindestens 40 Gew.-% Wasser enthält, und
b) 5 bis 70 Gew.-% einer von a) durch eine Phasengrenze getrennt vorliegenden transparenten Ölphase, die - bezogen auf ihr Gewicht - mindestens 5 Gew.-% eines flüchtigen linearen Silikons enthält.

Unter "flüssig" wird bevorzugt eine Viskosität der Phasen a) und b) von jeweils maximal 1000 mPas, vorzugsweise von maximal 750 mPas, mehr bevorzugt von maximal 500 mPas und insbesondere von maximal 250 mPas (jeweils gemessen bei 20°C mit einem Brookfield-Viskosimeter DV-II, Spindel 4 bei 20 UpM) verstanden.

Die beiden Phasen a) und b) des erfindungsgemäßen Haarkonditioniermittels sind bevorzugt transparent. Darunter wird verstanden, dass beide Phasen a) und b) im Ruhezustand bevorzugt einen NTU-Wert (Nephelometric Turbidity Unit) von maximal 100, vorzugsweise von maximal 75, mehr bevorzugt von maximal 50 und insbesondere von maximal 25 aufweisen.

Zur besseren Visualisierung kann eine der beiden Phasen a) oder b) mit einem kosmetisch akzeptablen Farbstoff angefärbt sein.

Alternativ können auch beide Phasen a) und b) unterschiedlich gefärbt vorliegen.

Im Ruhezustand liegen die beiden Phasen a) und b) bevorzugt durch eine Phasengrenze getrennt voneinander vor. Die Durchmischung der Phasen kann unmittelbar vor der Anwendung bevorzugt durch kräftiges Schütteln erreicht werden. Eine ausreichende Durchmischung lässt sich bevorzugt daran erkennen, dass nur noch eine Phase sichtbar ist, die transparent bis opak sein kann.

Vorzugsweise sollen die erfindungsgemäßen Haarkonditioniermittel versprühbar sein.

In versprühbarer Form eignen sich die erfindungsgemäßen Haarkonditioniermittel für die Applikation aus einem Pumpspender. Vorteilhaft an einer solchen Applikationsform ist die einfache, saubere und zeitsparende Handhabbarkeit, denn die Haarkonditioniermittel lassen sich aus einem geeigneten Pumpspender durch einfache Betätigung eines Pumpventils als feiner Sprühnebel verteilen und erreichen alle Bereiche der Haare.

Die Hände kommen auf diese Weise nicht mit dem Haarkonditioniermittel in Berührung und müssen nach der Anwendung des Mittels nicht gereinigt werden.

Für eine solche Applikationsform weisen die anwendungsbereiten (d.h. die durch Schütteln durchmischten) Haarkonditioniermittel bevorzugt eine Viskosität von maximal 1000 mPas, vorzugsweise von maximal 800 mPas, mehr bevorzugt von maximal 600 mPas und insbesondere von maximal 500 mPas (jeweils gemessen bei 20°C mit einem Brookfield-Viskosimeter DV-II, Spindel 4 bei 20 UpM) auf.

Ebenfalls bevorzugt für eine solche Applikationsform ist, dass die Haarkonditioniermittel frei von Treibmitteln sind.

Die Ölphase b) des erfindungsgemäßen Haarkonditioniermittels enthält - bezogen auf ihr Gewicht - bevorzugt 5 bis 60 Gew.-%, vorzugsweise 10 bis 70 Gew.-%, besonders bevorzugt 25 bis 80 Gew.-% und insbesondere 50 bis 99 Gew.-% mindestens eines Silikons der Formel (I), 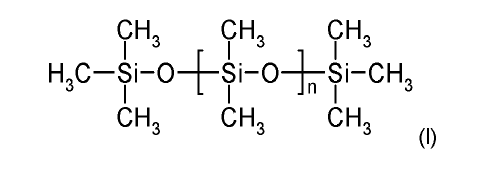 und/oder mindestens eines Silikons der Formel (II) 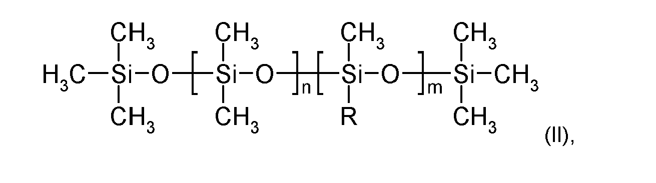 in der/denen

n jeweils für ganze Zahlen von 0 bis 8, m für ganze Zahlen von 0 bis 8 und R für eine C₂- C₈-Alkylgruppe, eine Arylgruppe oder eine C₁-C₈-Hydroxyalkylgruppe steht, wobei n + m für die Zahlen 0, 1, 2, 3, 4, 5, 6, 7 und 8 steht.

In einer bevorzugten Ausführungsform enthalten die erfindungsgemäßen Haarkonditioniermittel ein oder mehrere Silikon(e) der zuvor genannten Formeln (I) und/oder (II), in denen n jeweils für eine ganze Zahl von 0 bis 5 und insbesondere für die Zahlen 0, 1 oder 2, m für 0 oder 1 und R für eine C₂-C₄-Alkylgruppe oder eine Phenylgruppe steht.

Insbesondere bevorzugte erfindungsgemäße Haarkonditioniermittel enthalten Octamethyltrisiloxan, wie es beispielsweise von der Firma Dow unter der Bezeichnung "Dow Corning 200 Fluid 1 cSt" erhältlich ist.

In einer weiteren bevorzugten Ausführungsform können die erfindungsgemäßen Haarkonditioniermittel in der Ölphase b) - bezogen auf das Gewicht der Ölphase b) - weiterhin 0,01 bis 20 Gew.-%, vorzugsweise 0,025 bis 15 Gew.-%, besonders bevorzugt 0,05 bis 10 Gew.-% und insbesondere 0,1 bis 5 Gew.-% mindestens eines weiteren Öls, bevorzugt aus der Gruppe der pflanzlichen Öle, enthalten.

Geeignete pflanzliche Öle können ausgewählt sein aus Amaranthsamenöl, Aprikosenkernöl, Arganöl, Avocadoöl, Babassuöl, Baumwollsaatöl, Borretschsamenöl, Camelinaöl, Distelöl, Erdnußöl, Granatapfelkernöl, Grapefruitsamenöl, Hanföl, Hagebuttenkernöl, Haselnussöl, Holundersamenöl, Johannisbeersamenöl, Jojobaöl, Kakaobutter, Leinöl, Macadamianussöl, Maiskeimöl, Mandelöl, Marulaöl, Nachtkerzenöl, Olivenöl, Palmöl, Pfirsichkernöl, Rapsöl, Reisöl, Sanddornfruchtfleischöl, Sanddornkernöl, Sesamöl, Sheabutter, Sojaöl, Sonnenblumenöl, Traubenkernöl, Walnußöl oder Wildrosenöl.

Besonders bevorzugt sind Avocadoöl, Aprikosenkernöl, Hagebuttenkernöl, Jojobaöl, Mandelöl, Olivenöl, Pfirsichkernöl, Sonnenblumenöl und Traubenkernöl.

Bevorzugte erfindungsgemäße Haarkonditioniermittel enthalten die hydrophile Phase a) bevorzugt in Mengen von 35 bis 90 Gew.-% und die Ölphase b) bevorzugt in Mengen von 10 bis 65 Gew.-% - jeweils bezogen auf das Gewicht des anwendungsbereiten Haarkonditioniermittels.

Mengen von 40 bis 85 Gew.-% und insbesondere von 50 bis 80 Gew.-% der hydrophilen Phase a) sowie 15 bis 60 Gew.-% und insbesondere 20 bis 50 Gew.-% der Ölphase b) sind besonders bevorzugt.

Die hydrophile Phase a) enthält - bezogen auf ihr Gewicht - mindestens 40 Gew.-% Wasser. Bevorzugt sind erfindungsgemäße Haarkonditioniermittel, in denen die hydrophile Phase a) - bezogen auf ihr Gewicht - mindestens 45 Gew.-%, vorzugsweise mindestens 50 Gew.-%, besonders bevorzugt mindestens 55 Gew.-% und insbesondere mindestens 60 Gew.-% Wasser enthält.

Zur Beschleunigung der Phasentrennung ist es bevorzugt, wenn die erfindungsgemäßen Haarkonditioniermittel in der hydrophilen Phase a) weiterhin mindestens einen geradkettigen oder verzweigten C₁-C₆-Alkohol mit bis zu sechs Hydroxylgruppen im Molekül enthalten.

Geeignete Alkohole sind beispielsweise Ethanol, Propanol, Isopropanol, n-Butanol, Iso-butanol (2-Methy-1-propanol), 1-Pentanol, 2-Pentanol, 3-Pentanol, 1-Hexanol, 2-Hexanol, 3-Hexanol, 1,2-Propandiol, 1,2-Hexandiol, Glycerin und/oder Sorbitol, die sowohl einzeln als auch als Alkoholmischung in den erfindungsgemäßen Haarkonditioniermitteln eingesetzt werden können. Besonders bevorzugte Haarkonditioniermittel enthalten in der hydrophilen Phase a) - bezogen auf das Gewicht der hydrophilen Phase a) - höchstens 45 Gew.-%, bevorzugt höchstens 40 Gew.-% und insbesondere höchstens 35 Gew.-% Ethanol und/oder Isopropanol sowie höchstens 5 Gew.-%, bevorzugt höchstens 3 Gew.-% und insbesondere höchstens 2 Gew.-% Glycerin.

In einer besonders bevorzugten Ausführungsform enthalten die erfindungsgemäßen Haarkonditioniermittel bevorzugt
- 50 bis 80 Gew.-% der hydrophilen Phase a), die - bezogen auf ihr Gewicht - mindestens 60 Gew.-% Wasser sowie höchstens 35 Gew.-% Ethanol und/oder Isopropanol und höchstens 5 Gew.-% Glycerin enthält, und
- 20 bis 50 Gew.-% der Ölphase b), die - bezogen auf ihr Gewicht - 50 bis 99 Gew.-% mindestens eines Silikons der zuvor definierten Formeln (I) oder (II) sowie 0,1 bis 5 Gew.-% mindestens eines pflanzlichen Öls enthält.

Innerhalb dieser Ausführungsform ist es ganz besonders bevorzugt, wenn die Haarkonditioniermittel
- 50 bis 80 Gew.-% der hydrophilen Phase a), die - bezogen auf ihr Gewicht - mindestens 60 Gew.-% Wasser sowie höchstens 35 Gew.-% Ethanol und/oder Isopropanol und höchstens 5 Gew.-% Glycerin enthält, und
- 20 bis 50 Gew.-% der Ölphase b) enthalten, die - bezogen auf ihr Gewicht - 50 bis 99 Gew.-% Hexamethyldisiloxan, Octamethyltrisiloxan, Decamethyltetrasiloxan und/oder Phenyltrimethicon sowie 0,1 bis 5 Gew.-% Avocadoöl, Aprikosenkernöl, Hagebuttenkernöl, Jojobaöl, Mandelöl, Olivenöl, Pfirsichkernöl, Sonnenblumenöl und/oder Traubenkernöl enthält,
und insbesondere bevorzugt, wenn die Haarkonditioniermittel
- 50 bis 80 Gew.-% der hydrophilen Phase a), die - bezogen auf ihr Gewicht - mindestens 60 Gew.-% Wasser sowie höchstens 35 Gew.-% Ethanol und/oder Isopropanol und höchstens 5 Gew.-% Glycerin enthält, und
- 20 bis 50 Gew.-% der Ölphase b) enthalten, die - bezogen auf ihr Gewicht - 50 bis 99 Gew.-% Octamethyltrisiloxan sowie 0,1 bis 5 Gew.-% Avocadoöl, Aprikosenkernöl, Hagebuttenkernöl, Jojobaöl, Mandelöl, Olivenöl, Pfirsichkernöl, Sonnenblumenöl und/oder Traubenkernöl enthält.

Zur weiteren Steigerung der haarpflegenden Eigenschaften können die Haarkonditioniermittel in einer weiteren bevorzugten Ausführungsform in der hydrophilen Phase a) - bezogen auf das Gewicht der hydrophilen Phase a) - weiterhin 0,01 bis 7,5 Gew.-%, vorzugsweise 0,05 bis 5 Gew.-% und insbesondere 0,1 bis 3 Gew.-% mindestens eines linearen oder verzweigten, gesättigten oder ungesättigten physiologisch verträglichen C₁-C₂₄-Alkyltri(C₁-C₄)-alkylammoniumsalzes, Di-C₁-C₂₄-Alkyldi(C₁-C₄)-alkylammoniumsalzes oder Tri-C₁-C₂₄-Alkyl(C₁-C₄)-alkylammoniumsalzes und/oder 0,01 bis 7,5 Gew.-%, vorzugsweise 0,05 bis 5 Gew.-% und insbesondere 0,1 bis 3 Gew.-% mindestens eines primären, sekundären oder tertiären Amins, das mindestens eine Gruppe R-CO(NH)-(CH₂)ₙ- enthält, in der R für eine lineare oder verzweigte, gesättigte oder ungesättigte Alkylgruppe mit 7 bis 21 C-Atomen und n für eine ganze Zahl von 1 bis 4 steht, enthalten.

Besonders bevorzugte C₁-C₂₄-Alkyltri(C₁-C₄)-alkylammoniumsalze, Di-C₁-C₂₄-Alkyldi(C₁-C₄)-alkylammoniumsalze oder Tri-C₁C₂₄-Alkyl(C₁C₄)-alkylammoniumsalze sind Ammoniumhalogenide und/oder -methosulfate, insbesondere Chloride, Bromide und Methosulfate, wie beispielsweise Distearyldimethylammoniumchlorid, Lauryltrimethylammoniumchlorid, Lauryltrimethylbenzylammoniumchlorid, Tricetylmethylammoniumchlorid, Cetyltrimethylammoniumchlorid, Cetyltrimethylammoniumbromid, Cetyltrimethylammonium-methosulfat, Stearyltrimethylammoniumchlorid, Behenyltrimethylammoniumchlorid, Behenyltrimethylammoniumbromid und Behenyltrimethylammoniummethosulfat.

Ganz besonders bevorzugt sind Cetyltrimethylammonium- und Behenyltrimethylammoniumsalze und insbesondere Cetyltrimethylammoniumsalze, die als Anion ein Methosulfat- und/oder ein Chloridion enthalten.

Besonders bevorzugte primäre, sekundäre oder tertiäre Amine, die mindestens eine Gruppe R-CO(NH)-(CH₂)ₙ- enthalten, in der R für eine lineare oder verzweigte, gesättigte oder ungesättigte Alkylgruppe mit 7 bis 21 C-Atomen, und n für eine ganze Zahl von 1 bis 4 steht, sind bekannt unter der Bezeichnung "Amidoamin".

Geeignete Amidoamine können sowohl als solche, als auch (infolge einer Protonierung in entsprechend saurer Lösung) jeweils in der Form ihrer entsprechenden quaternären Verbindung in den Haarkonditioniermitteln vorliegen.

Bevorzugt sind die nicht-kationischen Amidoamine.

Besonders geeignete Amidoamine, welche gegebenenfalls quaternisiert sein können, sind beispielsweise Tego Amid^{®} S18 (Evonik; INCI-Bezeichnung: Stearamidopropyl Dimethylamine), Lexamine^{®} S 13 (Inolex; INCI-Bezeichnung: Stearamidopropyl Dimethylamine), Incromine^{®} SB (Croda; INCI-Bezeichnung: Stearamidopropyl Dimethylamine), Witcamine^{®} 100 (Witco, INCI-Bezeichnung: Cocamidopropyl Dimethylamine), Incromine^{®} BB (Croda, INCI-Bezeichnung: Behenamidopropyl Dimethylamine), Mackine^{®} 401 (McIntyre, INCI-Bezeichnung: Isostearylamidopropyl Dimethylamine) und andere Mackine-Typen sowie Adogen^{®} S18V (Witco, INCI-Bezeichnung: Stearylamidopropyl Dimethylamine).

Als permanent kationische Amidoamine können beispielsweise eingesetzt werden: Rewoquat^{®} RTM 50 (Witco Surfactants GmbH, INCI-Bezeichnung: Ricinoleamidopropyltrimonium Methosulfate), Empigen^{®} CSC (Albright&Wilson, INCI-Bezeichnung: Cocamidopropyltrimonium Chlorid), Swanol Lanoquat^{®} DES-50 (Nikko, INCI-Bezeichnung: Quatemium-33), Rewoquat^{®} UTM 50 (Witco Surfactants GmbH, Undecyleneamidopropyltrimonium Methosulfate).

Insbesondere bevorzugt ist Stearamidopropyl Dimethylamine.

In einer weiteren besonders bevorzugten Ausführungsform enthalten die erfindungsgemäßen Haarkonditioniermittel bevorzugt
a) 50 bis 80 Gew.-% der hydrophilen Phase a), die - bezogen auf ihr Gewicht-
   (i) mindestens 60 Gew.-% Wasser sowie höchstens 35 Gew.-% Ethanol und/oder Isopropanol und höchstens 2 Gew.-% Glycerin,
   (ii) 0,1 bis 3 Gew.-% eines C₁-C₂₄-Alkyltri(C₁-C₄)-alkylammonium-, Di-C₁-C₂₄-Alkyldi(C₁-C₄)-alkylammonium- oder Tri-C₁-C₂₄-Alkyl(C₁-C₄)-alkylammoniumsalzes und/oder
   (iii) 0,1 bis 3 Gew.-% eines primären, sekundären oder tertiären Amins, das mindestens eine Gruppe R-CO(NH)-(CH₂)ₙ- enthält, in der R für eine lineare oder verzweigte, gesättigte oder ungesättigte Alkylgruppe mit 7 bis 21 C-Atomen, und n für eine ganze Zahl von 1 bis 4 steht, enthält, und
b) 20 bis 50 Gew.-% der Ölphase b), die - bezogen auf ihr Gewicht - 50 bis 99 Gew.-% mindestens eines Silikons der zuvor definierten Formeln (I) oder (II) sowie 0,1 bis 5 Gew.-% mindestens eines pflanzlichen Öls enthält.

Innerhalb dieser Ausführungsform sind solche Haarkonditioniermittel ganz besonders bevorzugt, die
a) 50 bis 80 Gew.-% der hydrophilen Phase a), die - bezogen auf ihr Gewicht -
   (i) mindestens 60 Gew.-% Wasser sowie höchstens 35 Gew.-% Ethanol und/oder Isopropanol und höchstens 2 Gew.-% Glycerin,
   (ii) 0,1 bis 3 Gew.-% eines C₁-C₂₄-Alkyltri(C₁-C₄)-alkylammonium-, Di-C₁-C₂₄-Alkyldi(C₁-C₄)-alkylammonium- oder Tri-C₁-C₂₄-Alkyl(C₁-C₄)-alkylammoniumsalzes und/oder
   (iii) 0,1 bis 3 Gew.-% eines primären, sekundären oder tertiären Amins, das mindestens eine Gruppe R-CO(NH)-(CH₂)ₙ- enthält, in der R für eine lineare oder verzweigte, gesättigte oder ungesättigte Alkylgruppe mit 7 bis 21 C-Atomen, und n für eine ganze Zahl von 1 bis 4 steht, enthält, und
b) 20 bis 50 Gew.-% der Ölphase b) enthalten, die - bezogen auf ihr Gewicht - 50 bis 99 Gew.-% Hexamethyldisiloxan, Octamethyltrisiloxan, Decamethyltetrasiloxan und/oder Phenyltrimethicon sowie 0,1 bis 5 Gew.-% Avocadoöl, Aprikosenkernöl, Hagebuttenkernöl, Jojobaöl, Mandelöl, Olivenöl, Pfirsichkernöl, Sonnenblumenöl und/oder Traubenkernöl enthält.

Insbesondere bevorzugte Haarkonditioniermittel innerhalb dieser Ausführungsform enthalten
a) 50 bis 80 Gew.-% der hydrophilen Phase a), die - bezogen auf ihr Gewicht-
   (i) mindestens 60 Gew.-% Wasser sowie höchstens 35 Gew.-% Ethanol und/oder Isopropanol und höchstens 2 Gew.-% Glycerin,
   (ii) 0,1 bis 3 Gew.-% eines C₁-C₂₄-Alkyltri(C₁-C₄)-alkylammonium-, Di-C₁-C₂₄-Alkyldi(C₁-C₄)-alkylammonium- oder Tri-C₁-C₂₄-Alkyl(C₁-C₄)-alkylammoniumsalzes und/oder
   (iii) 0,1 bis 3 Gew.-% eines primären, sekundären oder tertiären Amins, das mindestens eine Gruppe R-CO(NH)-(CH₂)ₙ- enthält, in der R für eine lineare oder verzweigte, gesättigte oder ungesättigte Alkylgruppe mit 7 bis 21 C-Atomen, und n für eine ganze Zahl von 1 bis 4 steht, enthält, und
b) 20 bis 50 Gew.-% der Ölphase b), die - bezogen auf ihr Gewicht - 50 bis 99 Gew.-% Octamethyltrisiloxan sowie 0,1 bis 5 Gew.-% Avocadoöl, Aprikosenkernöl, Hagebuttenkernöl, Jojobaöl, Mandelöl, Olivenöl, Pfirsichkernöl, Sonnenblumenöl und/oder Traubenkernöl enthält.

Als weitere fakultative Komponenten zur Unterstützung der haarpflegenden Wirkung können die erfindungsgemäßen Haarkonditioniermittel bevorzugt mindestens einen Wirkstoff aus der Gruppe der Vitamine sowie der Provitamine, Vitaminderivate oder Vitaminvorstufen, der Proteinhydrolysate sowie der quaternisierten Proteinhydrolysate und/oder der kationischen Polymere enthalten.
Diese Wirkstoffe sind bevorzugt in den hydrophilen Phase a) des Haarkonditioniermittels enthalten.

Geeignete Vitamine, Provitamine und Vitaminvorstufen sowie deren Derivate sind den Gruppen A, B, C, E, F und H zuzuordnen.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen beispielsweise Vitamin A-Säure und deren Ester, Vitamin A-Aldehyd und Vitamin A-Alkohol sowie dessen Ester wie das Palmitat und das Acetat in Betracht. Die Haarkonditioniermittel können die Vitamin A-Komponente bevorzugt in Mengen von 0,05-1 Gew.-%, bezogen auf das gesamte Mittel, enthalten.
Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Bevorzugt ist das Nicotinsäureamid, das in den Haarkonditioniermitteln bevorzugt in Mengen von 0,05 bis 1 Gew.-%, bezogen auf das gesamte Mittel, enthalten sein kann.
- Vitamin B₅ (Pantothensäure, Panthenol und Pantolacton). Im Rahmen dieser Gruppe wird bevorzugt das Panthenol und/oder Pantolacton eingesetzt. Einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie kationische Panthenolderivate. Die genannten Verbindungen des Vitamin B₅-Typs können in den Haarkonditioniermitteln bevorzugt in Mengen von 0,05 - 10 Gew.-%, bezogen auf das gesamte Mittel, enthalten sein. Mengen von 0,1 - 5 Gew.-% sind besonders bevorzugt.
- Vitamin B₆ (Pyridoxin sowie Pyridoxamin und Pyridoxal).
   Vitamin C (Ascorbinsäure). Vitamin C kann in den Haarkonditioniermitteln bevorzugt in Mengen von 0,1 bis 3 Gew.-%, bezogen auf das gesamte Mittel, eingesetzt werden. Die Verwendung in Form des Palmitinsäureesters, der Glucoside oder Phosphate kann bevorzugt sein. Die Verwendung in Kombination mit Tocopherolen kann ebenfalls bevorzugt sein.
   Vitamin E (Tocopherole, insbesondere α-Tocopherol). Tocopherol und seine Derivate, worunter insbesondere die Ester wie das Acetat, das Nicotinat, das Phosphat und das Succinat fallen, können in den Haarkonditioniermitteln bevorzugt in Mengen von 0,05-1 Gew.-%, bezogen auf das gesamte Mittel, enthalten sein.
   Vitamin F. Unter dem Begriff "Vitamin F" werden üblicherweise essentielle Fettsäuren, insbesondere Linolsäure, Linolensäure und Arachidonsäure, verstanden.
   Vitamin H. Als Vitamin H wird die Verbindung (3aS,4S, 6aR)-2-Oxohexahydrothienol[3,4-d]-imidazol-4-valeriansäure bezeichnet, für die sich aber inzwischen der Trivialname Biotin durchgesetzt hat. Biotin kann in den Haarkonditioniermitteln bevorzugt in Mengen von 0,0001 bis 1,0 Gew.-%, insbesondere in Mengen von 0,001 bis 0,01 Gew.-% enthalten sein.

Bevorzugt können die erfindungsgemäßen Haarkonditioniermittel Vitamine, Provitamine und Vitaminvorstufen aus den Gruppen A, B, E und H enthalten. Panthenol, Pantolacton, Pyridoxin und seine Derivate sowie Nicotinsäureamid und Biotin sind besonders bevorzugt und können den erfindungsgemäßen Haarkonditioniermitteln sowohl einzeln, als auch in ihrer Kombination zugesetzt werden.

Geeignete Proteinhydrolysate und/oder quaternisierte Proteinhydrolysate können sowohl pflanzlichen als auch tierischen, marinen oder synthetischen Ursprungs sein.

Geeignete Proteinhydrolysate und/oder quaternisierte Proteinhydrolysate können die Kräftigung der Haarstruktur unterstützen und das Austrocknen der Haare verhindern.

Bevorzugte tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.

Bevorzugte pflanzliche Proteinhydrolysate sind beispielsweise Soja-, Mandel-, Erbsen-, Kartoffel-und Weizenproteinhydrolysate. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Zu den geeigneten Proteinhydrolysaten maritimen Ursprunges zählen beispielsweise Kollagenhydrolysate von Fischen oder Algen sowie Proteinhydrolysate von Muscheln bzw. Perlenhydrolysate. Beispiele für erfindungsgemäße Perlenhydrolysate sind die Handelsprodukte Pearl Protein Extract BG^{®} oder Crodarom^{®} Pearl.

Geeignete kationisierte Proteinhydrolysate können ebenfalls tierischen, pflanzlichen oder maritimen Ursprungs sein.

Die Quaternisierung der Proteinhydrolysate oder der Aminosäuren kann mit quarternären Ammoniumsalzen wie beispielsweise N,N-Dimethyl-N-(n-Alkyl)-N-(2-hydroxy-3-chloro-n-propyl)-ammoniumhalogeniden durchgeführt werden. Darüber hinaus können die kationischen Proteinhydrolysate auch noch weiter derivatisiert sein.

Als typische Beispiele für besonders geeignete kationische Proteinhydrolysate und/oder deren Derivate seien die unter den INCI - Bezeichnungen bekannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed keratin, Hydroxypropyltrimonium Hydrolyzed Rice Bran Protein, Hydroxyproypltrimonium Hydrolyzed Silk, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Silk, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Silk, Quaternium-79 Hydrolyzed Soy Protein, Quaternium-79 Hydrolyzed Wheat Protein.

Besonders bevorzugt für die Verwendung in den erfindungsgemäßen Haarkonditioniermitteln sind Keratin-, Seiden-, Milcheiweiß-, Weizen- und/oder Sojaproteinhydrolysate, die quaternisiert sein können. Insbesondere bevorzugt sind Keratin- und/oder Weizen- und/oder Seidenproteinhydrolysate, die quaternisiert sein können.

Die - gegebenenfalls quaternisierten - Proteinhydrolysate können in den erfindungsgemäßen Haarkonditioniermitteln sowohl einzeln, als auch als Mischung eingesetzt werden.

Es kann für einige Ausführungsformen der Erfindung von Vorteil sein, wenn die Haarkonditioniermittel mindestens ein Proteinhydrolysat tierischen oder pflanzlichen Ursprungs sowie mindestens ein quaternisiertes Proteinhydrolysat tierischen oder pflanzlichen Ursprungs enthalten.

Die Proteinhydrolysate und/oder quaternisierten Proteinhydrolysate können in den Haarkonditioniermitteln - bezogen auf deren Gesamtgewicht - bevorzugt in Mengen von 0,01 bis 15 Gew.-%, vorzugsweise von 0,05 bis 10 Gew.-% und insbesondere in Mengen von 0,1 bis 5 Gew.-% enthalten sein.

Geeignete kationische Polymere, die in den erfindungsgemäßen Haarkonditioniermitteln eingesetzt werden können, sind bekannt unter der INCI-Bezeichnung "Polyquaternium".

Besonders geeignete kationische Polymere sind Polyquaternium-6, Polyquaternium-7, Polyquaternium-10, Polyquaternium-11, Polyquaternium-16, Polyquaternium-22, Polyquaternium-24, Polyquaternium-28, Polyquaternium-37, Polyquaternium-67, Polyquaternium-74 und Polyquaternium-89.

Insbesondere geeignet sind kationische Polymere, die sich von Polymeren natürlichen Ursprungs wie Cellulose-, Stärke- oder Guarpolymeren ableiten.

Kationische Cellulosederivate werden beispielsweise von der Firma Amerchol unter der Bezeichnung Polymer JR^{®} und kationische Guarpolymere von der Firma Rhöne-Poulenc oder Hercules unter den Bezeichnungen Jaguar^{®} oder N-Hance^{®} angeboten.

Weitere geeignete kationische Polymere natürlichen Ursprungs sind beispielsweise kationische Chitosane, die beispielsweise unter den Bezeichnungen "Kytamer" oder "Hydagen" im Handel erhältlich sind.

Weiterhin geeignet sind die unter der INCI-Bezeichnung "Quaternium" bekannten kationischen Polymere wie Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-82, Quaternium-83 und/oder Quaternium-84.

Weitere geeignete kationische Polymere sind beispielsweise
- kationische Alkylpolyglycoside,
- kationisierter Honig, beispielsweise das Handelsprodukt Honeyquat^{®} 50,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
- sowie die unter den Bezeichnungen Polyquaternium-2, Polyquaternium-17, Polyquaternium-18 und Polyquaternium-27 bekannten Polymere mit quartären Stickstoffatomen in der Polymerhauptkette,
- Vinylpyrrolidon-Vinylcaprolactam-Acrylat-Terpolymere, wie sie mit Acrylsäureestern und Acrylsäureamiden als dritter Monomerbaustein im Handel beispielsweise unter der Bezeichnung Aquaflex^{®} SF 40 angeboten werden.

Die kationischen Polymere können in den erfindungsgemäßen Haarkonditioniermitteln einzeln oder als Mischung bevorzugt in Mengen von 0,01 bis 10 Gew.-%, vorzugsweise von 0,05 bis 5 Gew.-% und insbesondere in Mengen von 0,1 bis 2 Gew.-% eingesetzt werden, wobei sich die Mengen auf das Gewicht des Haarkonditioniermittels beziehen.

In einer weiteren besonders bevorzugten Ausführungsform enthalten die erfindungsgemäßen Haarkonditioniermittel
a) 50 bis 80 Gew.-% der hydrophilen Phase a), die - bezogen auf ihr Gewicht-
   (i) mindestens 60 Gew.-% Wasser sowie höchstens 35 Gew.-% Ethanol und/oder Isopropanol und höchstens 2 Gew.-% Glycerin,
   (ii) 0,1 bis 3 Gew.-% eines C₁-C₂₄-Alkyltri(C₁-C₄)-alkylammonium-, Di-C₁-C₂₄-Alkyldi(C₁-C₄)-alkylammonium- oder Tri-C₁-C₂₄-Alkyl(C₁-C₄)-alkylammoniumsalzes und/oder
   (iii) 0,1 bis 3 Gew.-% eines primären, sekundären oder tertiären Amins, das mindestens eine Gruppe R-CO(NH)-(CH₂)ₙ- enthält, in der R für eine lineare oder verzweigte, gesättigte oder ungesättigte Alkylgruppe mit 7 bis 21 C-Atomen, und n für eine ganze Zahl von 1 bis 4 steht, und/oder
   (iv) 0,05 bis 1 Gew.-% Panthenol, Pantolacton, Pyridoxin und seine Derivate, Nicotinsäureamid und/oder Biotin und/oder
   (v) 0,1 bis 5 Gew.-% eines Proteinhydrolysats und/oder quaternisierten Proteinhydrolysats tierischen oder pflanzlichen Ursprungs und/oder
   (vi) 0,1 bis 2 Gew.-% eines zuvor definierten kationischen Polymeren enthält, und
b) 20 bis 50 Gew.-% der Ölphase b), die - bezogen auf ihr Gewicht - 50 bis 99 Gew.-% mindestens eines Silikons der zuvor definierten Formeln (I) oder (II) sowie 0,1 bis 5 Gew.-% eines pflanzlichen Öls enthält.

Innerhalb dieser besonders bevorzugten Ausführungsform ist es mehr bevorzugt, wenn die Haarkonditioniermittel
a) 50 bis 80 Gew.-% der hydrophilen Phase a), die - bezogen auf ihr Gewicht -
   (i) mindestens 60 Gew.-% Wasser sowie höchstens 35 Gew.-% Ethanol und/oder Isopropanol und höchstens 2 Gew.-% Glycerin,
   (ii) 0,1 bis 3 Gew.-% eines C₁-C₂₄-Alkyltri(C₁-C₄)-alkylammonium-, Di-C₁-C₂₄-Alkyldi(C₁-C₄)-alkylammonium- oder Tri-C₁-C₂₄-Alkyl(C₁-C₄)-alkylammoniumsalzes und
   (iii) 0,1 bis 3 Gew.-% eines primären, sekundären oder tertiären Amins, das mindestens eine Gruppe R-CO(NH)-(CH₂)ₙ- enthält, in der R für eine lineare oder verzweigte, gesättigte oder ungesättigte Alkylgruppe mit 7 bis 21 C-Atomen, und n für eine ganze Zahl von 1 bis 4 steht, und
   (iv) 0,05 bis 1 Gew.-% Panthenol, Pantolacton, Pyridoxin und seine Derivate, Nicotinsäureamid und/oder Biotin und/oder
   (v) 0,1 bis 5 Gew.-% eines Proteinhydrolysats und/oder quaternisierten Proteinhydrolysats tierischen oder pflanzlichen Ursprungs und/oder
   (vi) 0,1 bis 2 Gew.-% eines zuvor definierten kationischen Polymeren enthält, und
b) 20 bis 50 Gew.-% der Ölphase b) enthalten, die - bezogen auf ihr Gewicht - 50 bis 99 Gew.-% Hexamethyldisiloxan, Octamethyltrisiloxan, Decamethyltetrasiloxan und/oder

Phenyltrimethicon sowie 0,1 bis 5 Gew.-% Avocadoöl, Aprikosenkernöl, Hagebuttenkernöl, Jojobaöl, Mandelöl, Olivenöl, Pfirsichkernöl, Sonnenblumenöl und/oder Traubenkernöl enthält.

Insbesondere bevorzugt innerhalb dieser Ausführungsform sind Haarkonditioniermittel, die
a) 50 bis 80 Gew.-% der hydrophilen Phase a), die - bezogen auf ihr Gewicht -
   (i) mindestens 60 Gew.-% Wasser sowie höchstens 35 Gew.-% Ethanol und/oder Isopropanol und höchstens 2 Gew.-% Glycerin,
   (ii) 0,1 bis 3 Gew.-% eines Cetyltrimethylammonium- oder Behenyltrimethylammoniumsalzes,
   (iii) 0,1 bis 3 Gew.-% Stearamidopropyldimethylamin,
   (iv) 0,05 bis 1 Gew.-% Panthenol, Pantolacton, Nicotinsäureamid und/oder Biotin,
   (v) 0,1 bis 5 Gew.-% eines Proteinhydrolysats und eines quaternisierten Proteinhydrolysats tierischen oder pflanzlichen Ursprungs enthält, und
b) 20 bis 50 Gew.-% der Ölphase b) enthalten, die - bezogen auf ihr Gewicht - 50 bis 99 Gew.-% Octamethyltrisiloxan sowie 0,1 bis 5 Gew.-% Avocadoöl, Aprikosenkernöl, Hagebuttenkernöl, Jojobaöl, Mandelöl, Olivenöl, Pfirsichkernöl, Sonnenblumenöl und Traubenkernöl enthält.

Ferner können die Haarkonditioniermittel weitere Wirk-, Hilfs- und Zusatzstoffe enthalten, wie beispielsweise
- Strukturanten wie Maleinsäure und Milchsäure,
- Parfümöle, Dimethylisosorbid und Cyclodextrine,
- Farbstoffe zum Anfärben des Mittels,
- Antischuppenwirkstoffe wie Piroctone Olamine, Zink Omadine und Climbazol,
- Cholesterin,
- Komplexbildner wie EDTA, NTA, β-Alanindiessigsäure und Phosphonsäuren,
- Pigmente,
- Antioxidantien und/oder UV-Filter,
- amphotere, anionische und/oder anionische Polymere,
- kationische, amphotere und/oder nichtionische Tenside,
- weitere Fett- und/oder Ölkomponenten wie Silikonöle, Fettsäuren, Fettalkohole und deren Derivate,
- Carbonsäuren wie Milchsäure, Zitronensäure, Äpfelsäure, Glycolsäure,
- Pflanzenextrakte,
- Penetrationshilfsmittel und/oder Quellmittel wie Harnstoff, Hydantoin und/oder deren Derivate.

Die erfindungsgemäßen Haarkonditioniermittel weisen bevorzugt einen pH-Wert im Bereich von 2,5 bis 7,5, bevorzugt von 3 bis 6 und insbesondere von 4 bis 5 auf.

Neben einem ästhetischen Erscheinungsbild haben die zweiphasigen transparenten Haarkonditioniermittel den Vorteil, dass sie leicht in der Handhabung und einfach in ihrer Herstellung sind.

Die spezielle Wirkstoffkombination in den Haarkonditioniermitteln gewährleistet eine schnelle Durchmischung der beiden Phasen vor der Anwendung und eine schnelle Entmischung der beiden Phasen nach der Anwendung, wobei sowohl auf den Einsatz von Cyclomethiconen als auch auf den Einsatz von Kohlenwasserstoffen verzichtet werden kann.

Die hydrophile Phase ist überwiegend wässrig, was den Bedürfnissen der Verbraucher entgegenkommt.

Die erfindungsgemäßen Haarkonditioniermittel eignen sich darüber hinaus besonders für die Pflege der Haare und verleihen den Haaren eine verbesserte Kämmbarkeit und mehr Glanz.

Die erfindungsgemäßen Haarkonditioniermittel sind versprühbar und eignen sich für die Applikation aus einem Pumpspender. Dadurch sind sie einfach in der Handhabung und effektiv in ihrer Wirkung, denn der aus dem Pumpspender austretende feine Sprühnebel erreicht alle Haarbereiche.

Ein zweiter Gegenstand der Erfindung ist ein Verfahren zur Konditionierung von Haaren, bei dem ein erfindungsgemäßes Haarkonditioniermittel nach kräftigem Schütteln auf die nassen oder trockenen Haare gesprüht, und entweder nach einer Einwirkungszeit von 5 Sekunden bis 5 Minuten wieder ausgespült, oder bis zur nächsten Haarreinigung auf den Haaren belassen wird.

### Beispiele:

Non-Aerosol-Spühkuren:

| | 1 (Gew.-%) | 2 (Gew.-%) |
|---|---|---|
| Octamethyltrisiloxan 1cs | 22 | 24 |
| Mandelöl | 1 | - |
| Sonnenblumenöl | - | 0,4 |
| Stearamidopropyldimethylamine | 1,5 | 1 |
| Cetrimoniumchlorid | 0,25 | 0,3 |
| Keratinhydrolysat | - | 0,1 |
| Pantolacton | 0,1 | - |
| Ethanol | 25 | 20 |
| Glycerin | 2 | 1 |
| Wasser | ad 100 | ad 100 |

| | 3 (Gew.-%) | 4 (Gew.-%) |
|---|---|---|
| Octamethyltrisiloxan 1cs | 19 | 22 |
| Aprikosenkernöl | 1 | 0,5 |
| Sonnenblumenöl | - | 0,2 |
| Stearamidopropyldimethylamine | 1,5 | 0,75 |
| Cetrimoniumchlorid | 0,1 | 0,15 |
| Keratinhydrolysat | 0,1 | 0,1 |
| Cocodimonium Hydroxypropyl Hydrolyzed Keratin | 0,05 | - |
| Seidenproteinhydrolysat | - | 0,05 |
| Pantolacton | 0,05 | 0,1 |
| Panthenol | 0,1 | - |
| Nicotinsäureamid | - | 0,005 |
| Ethanol | 25 | 20 |
| Glycerin | 1,5 | 1 |
| Wasser | ad 100 | ad 100 |

| | 5 (Gew.-%) | 6 (Gew.-%) |
|---|---|---|
| Octamethyltrisiloxan 1cs | 19,5 | 20 |
| Aprikosenkernöl | 0,5 | - |
| Jojobaöl | - | 3 |
| Stearamidopropyldimethylamine | 1 | - |
| Cocamidopropyl Dimethylamine | - | 1,5 |
| Cetrimoniumchlorid | 0,1 | - |
| Behentrimethylammoniumchlorid | - | 0,15 |
| Keratinhydrolysat | 0,1 | 0,1 |
| Cocodimonium Hydroxypropyl Hydrolyzed Keratin | 0,05 | - |
| Seidenproteinhydrolysat | 0,1 | 0,05 |
| Pantolacton | 0,05 | - |
| Panthenol | 0,1 | - |
| Nicotinsäureamid | - | 0,25 |
| PEG-7 Glyceryl Cocoate | 1,5 | 1 |
| Ethanol | 25 | 25 |
| Glycerin | 1,5 | 0,5 |
| Wasser | ad 100 | ad 100 |

| | 7 (Gew.-%) | 8 (Gew.-%) |
|---|---|---|
| Hexamethyldisiloxan | 19,5 | 20 |
| Aprikosenkernöl | 0,5 | - |
| Avocadoöl | - | 1 |
| Stearamidopropyldimethylamine | 1,5 | 1 |
| Behentrimethylammoniumchlorid | 0,25 | 0,1 |
| Keratinhydrolysat | 0,1 | 0,1 |
| Seidenproteinhydrolysat | | 0,1 |
| Panthenol | 0,1 | - |
| Biotin | | 0,05 |
| Ethanol | 22 | 25 |
| Glycerin | 2 | 1,5 |
| Wasser | ad 100 | ad 100 |

| | 9 (Gew.-%) | 10 (Gew.-%) |
|---|---|---|
| Decamethyltetrasiloxan | 18 | 20 |
| Aprikosenkernöl | 2 | - |
| Sonnenblumenöl | - | 0,5 |
| Cocamidopropyl Dimethylamine | 2 | 1,5 |
| Behentrimethylammoniumchlorid | 0,05 | 0,1 |
| Cetrimoniumchlorid | 0,1 | - |
| Keratinhydrolysat | 0,15 | 0,1 |
| Lauryldimonium Hydroxypropyl Hydrolyzed Silk | 0,1 | - |
| Hydroxypropyltrimonium Hydrolyzed Wheat Protein | - | 0,05 |
| Nicotinsäureamid | 0,1 | - |
| Pantolacton | 0,1 | 0,25 |
| PEG-7 Glyceryl Cocoate | 1,5 | 1 |
| Isopropanol | 25 | 20 |
| Glycerin | 1 | 1,75 |
| Wasser | ad 100 | ad 100 |

| | 11 (Gew.-%) | 12 (Gew.-%) |
|---|---|---|
| Phenyltrimethicon | 19,5 | 22 |
| Aprikosenkernöl | 0,5 | - |
| Avocadoöl | 1 | 0,5 |
| Behenamidopropyl Dimethylamine | 2 | 1,5 |
| Cetrimoniumchlorid | 0,1 | - |
| Seidenproteinhydrolysat | 0,15 | - |
| Lauryldimonium Hydroxypropyl Hydrolyzed Silk | 0,1 | 0,05 |
| Keratinhydrolysat | 0,05 | 0,1 |
| Panthenol | 0,1 | 0,2 |
| Ethanol | 22,5 | 25 |
| Glycerin | 0,5 | 1 |
| Wasser | ad 100 | ad 100 |

| | 13 (Gew.-%) | 14 (Gew.-%) |
|---|---|---|
| Octamethyltrisiloxan 1cs | 17,5 | 20 |
| Hexamethyldisiloxan | 2,5 | - |
| Decamethyltetrasiloxan | - | 5 |
| Aprikosenkernöl | 0,5 | - |
| Jojobaöl | 0,5 | 1 |
| Stearamidopropyldimethylamine | 1 | - |
| Cocamidopropyl Dimethylamine | - | 1,5 |
| Cetrimoniumchlorid | 0,1 | - |
| Behentrimethylammoniumchlorid | - | 0,15 |
| Keratinhydrolysat | 0,1 | 0,1 |
| Cocodimonium Hydroxypropyl Hydrolyzed Keratin | 0,05 | 0,1 |
| Seidenproteinhydrolysat | 0,1 | 0,05 |
| Pantolacton | 0,05 | - |
| Panthenol | 0,1 | - |
| Nicotinsäureamid | - | 0,25 |
| PEG-7 Glyceryl Cocoate | 1,5 | 1 |
| Ethanol | 25 | 25 |
| Glycerin | 1,5 | 0,5 |
| Wasser | ad 100 | ad 100 |

Die Haarkonditioniermittel der Beispiele 1 bis 14 werden hergestellt, indem die Komponenten der Ölphase (lineare(s) Silikon(e) und pflanzliche(s) Öl(e)) und der hydrophilen Phase jeweils separat vermischt werden, wobei jeweils transparente Lösungen entstehen. Die hydrophilen Phasen werden mit den jeweiligen Ölphasen während des Abfüllvorgangs in einem handelsüblichen Pumpspender vermischt. Im Ruhezustand trennen sie sich schnell in zwei separate transparente Phasen auf.

Für die Anwendung werden die Mittel kräftig geschüttelt und unmittelbar danach auf die nassen oder trockenen - vorzugsweise gereinigten - Haare gesprüht, wo sie bevorzugt bis zur nächsten Haarreinigung verbleiben.

## Patentansprüche

1. Flüssiges zweiphasiges Haarkonditioniermittel, enthaltend - bezogen auf das Gewicht des anwendungsbereiten Mittels -
a) 30 bis 95 Gew.-% einer transparenten hydrophilen Phase, die - bezogen auf ihr Gewicht - mindestens 40 Gew.-% Wasser enthält, und
b) 5 bis 70 Gew.-% einer von a) durch eine Phasengrenze getrennt vorliegenden transparenten Ölphase, die - bezogen auf ihr Gewicht - mindestens 5 Gew.-% eines flüchtigen linearen Silikons enthält.

2. Haarkonditioniermittel gemäß Anspruch 1, **dadurch gekennzeichnet, dass** die Ölphase b) - bezogen auf ihr Gewicht - 5 bis 60 Gew.-%, vorzugsweise 10 bis 70 Gew.-%, besonders bevorzugt 25 bis 80 Gew.-% und insbesondere 50 bis 99 Gew.-% mindestens eines Silikons der Formel (I) 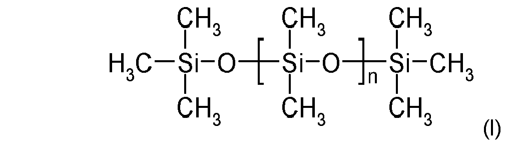 und/oder mindestes eines Silikons der Formel (II) 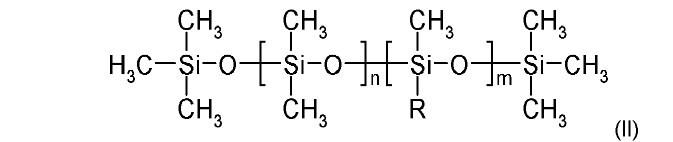 enthält, in der/denen
n jeweils für ganze Zahlen von 0 bis 8, m für ganze Zahlen von 0 bis 8 und R für eine C₂-C₈-Alkylgruppe, eine Arylgruppe oder eine C₁-C₈-Hydroxyalkylgruppe steht, wobei n + m für die Zahlen 0, 1, 2, 3, 4, 5, 6, 7 und 8 steht.

3. Haarkonditioniermittel gemäß Anspruch 2, **dadurch gekennzeichnet, dass** n jeweils für eine ganze Zahl von 0 bis 5, insbesondere für 0, 1 oder 2, m für 0 oder 1 und R für eine C₂-C₄-Alkylgruppe oder eine Phenylgruppe steht.

4. Haarkonditioniermittel gemäß einem der Ansprüche 1 bis 3, **dadurch gekennzeichnet, dass** die Ölphase b) Octamethyltrisiloxan enthält.

5. Haarkonditioniermittel gemäß einem der Ansprüche 1 bis 4, **dadurch gekennzeichnet, dass** die Ölphase b) - bezogen auf ihr Gewicht - 0,01 bis 20 Gew.-%, vorzugsweise 0,025 bis 15 Gew.-%, besonders bevorzugt 0,05 bis 10 Gew.-% und insbesondere 0,1 bis 5 Gew.-% mindestens eines weiteren Öls, bevorzugt ausgewählt aus der Gruppe der pflanzlichen Öle, enthält.

6. Haarkonditioniermittel nach einem der Ansprüche 1 bis 5, **dadurch gekennzeichnet, dass** es - bezogen auf sein Gewicht - 35 bis 90 Gew.-% der hydrophile Phase a) und 10 bis 65 Gew.-% der Ölphase b), vorzugsweise 40 bis 85 Gew.-% der hydrophile Phase a) und 15 bis 60 Gew.-% der Ölphase b) und insbesondere 50 bis 80 Gew.-% hydrophile Phase a) und 20 bis 50 Gew.-% der Ölphase b) enthält.

7. Haarkonditioniermittel nach einem der Ansprüche 1 bis 6, **dadurch gekennzeichnet, dass** die hydrophile Phase a) - bezogen auf ihr Gewicht - mindestens 45 Gew.-%, vorzugsweise mindestens 50 Gew.-%, besonders bevorzugt mindestens 55 Gew.-% und insbesondere mindestens 60 Gew.-% Wasser enthält.

8. Haarkonditioniermittel nach einem der Ansprüche 1 bis 7, **dadurch gekennzeichnet, dass** die hydrophile Phase a) - bezogen auf ihr Gewicht - höchstens 45 Gew.-%, vorzugsweise höchstens 40 Gew.-% und insbesondere höchstens 35 Gew.-% Ethanol und/oder Isopropanol enthält.

9. Haarkonditioniermittel einem der Ansprüche 1 bis 8, **dadurch gekennzeichnet, dass** die hydrophile Phase a) - bezogen auf ihr Gewicht - 0,01 bis 7,5 Gew.-%, vorzugsweise 0,05 bis 5 Gew.-% und insbesondere 0,1 bis 3 Gew.-% mindestens eines linearen oder verzweigten, gesättigten oder ungesättigten physiologisch verträglichen C₁-C₂₄-Alkyltri(C₁-C₄)-alkylammoniumsalzes, Di-C₁-C₂₄-Alkyldi(C₁-C₄)-alkylammoniumsalzes oder Tri-C₁-C₂₄-Alkyl(C₁-C₄)-alkylammoniumsalzes und/oder 0,01 bis 7,5 Gew.-%, vorzugsweise 0,05 bis 5 Gew.-% und insbesondere 0,1 bis 3 Gew.-% mindestens eines primären, sekundären oder tertiären Amins, das mindestens eine Gruppe R-CO(NH)-(CH₂)ₙ- enthält, in der R für eine lineare oder verzweigte, gesättigte oder ungesättigte Alkylgruppe mit 7 bis 21 C-Atomen und n für eine ganze Zahl von 1 bis 4 steht, enthält.

10. Verfahren zur Konditionierung von Haaren, **dadurch gekennzeichnet, dass** ein Mittel nach einem der Ansprüche 1 bis 9 nach kräftigem Schütteln auf die nassen oder trockenen Haare gesprüht, und entweder nach einer Einwirkungszeit von 5 Sekunden bis 5 Minuten wieder ausgespült, oder bis zur nächsten Haarreinigung auf den Haaren belassen wird.
